# EUROPEAN PATENT APPLICATION

(11) **EP 4 722 873 A1**
(43) Date of publication of application: **08.04.2026**
(21) Application number: 25206100.7
(22) Date of filing: 01.10.2025
(51) Int. Cl.: G06F 3/0481, G06F 3/0484

(54) **SYSTEMS AND METHODS FOR POWERING ON A CONTROLLER DEVICE BY A MEDICAL DISPLAY DEVICE IN A MEDICAL ENVIRONMENT**

(30) Priority: 02.10.2024 US 202463702428 P
(71) Applicant: Stryker Corporation, Portage, MI 49002 (US)
(72) Inventor: MISTRY, Neel, Portage, 49002 (US); HEREFORD, Eric Alexander, Portage, 49002 (US); SIMONS, Kyle Jason, Portage, 49002 (US)
(74) Representative: V.O.

(57) **Abstract**

Disclosed are systems and methods for powering on a controller device using a medical display device. In some aspects, the operating room may be configured with a controller device located outside of or in a hard-to-access location within the operating room. Having the ability to remotely power on a controller device may reduce/eliminate the need to have a medical operator leave the operating room and/or have an IT operator/on-site specialist power on the controller device. The medical display device may not receive one or more signals (e.g., video signals, image signals) from the controller device when the controller device is off or in standby mode. In response, the medical display device may provide an input element on its touch screen, and a medical operator may touch the input element to remotely power on the controller device.

## Description

### CROSS-REFERENCE TO RELATED APPLICATION

This application claims the benefit of U.S. Provisional Application No. 63/702,428, filed October 2, 2024, the entire contents of which is incorporated herein by reference.

### FIELD

The present invention relates to powering on a controller device by a medical display device, particularly for allowing a medical operator to be able to conveniently and efficiently power on the controller device.

### BACKGROUND

A medical procedure (e.g., surgery) generally involves an operating room that comprises medical devices coupled throughout the operating room using a routing system. Example medical devices may include audio devices (e.g., speakers, microphones), video devices (e.g., medical display and/or touch screen devices, cameras), and other types of devices (e.g., surgical lights, computers, a controller device, server, user interface device). The operating room may be configured such that one or more medical devices, such as a routing system and a controller device, are located outside of, or in a hard-to-access location within, the operating room.

In some instances, a medical operator may power down a controller device when the operating room is not used for a long period of time, such as at the end of the day, and the medical operator may power on the controller device once use of the operating room resumes. In some instances, a controller device may unexpectedly power off, such as when power to the operating room has been lost. When a medical device is located outside of the operating room, a medical operator may have to leave the operating room in order to power on the controller device. The medical operator may alternatively call for an information technology (IT) operator or an on-site specialist to power on the controller device. However, leaving the operating room or calling an IT operator/on-site specialist may be problematic as this may result in risk of unsanitary conditions and/or taking up extra time. It may be desirable to avoid or reduce the need for a medical operator to leave an operating room or the need to call an IT operator/on-site specialist by providing a convenient and efficient way to power on a controller device.

### SUMMARY

According to various aspects, systems and methods include reducing or eliminating the need to have a medical operator leave the operating room and/or the need to call an IT operator/on-site specialist in order to power on a controller device. Examples of the disclosure provide the ability to power on a controller device remotely using, *e.g.,* a medical display device (e.g., a medical touch screen device). The medical display device may provide an input element (e.g., display a graphical user interface (GUI) element on its touch screen) when the controller device is powered off or in a standby mode. The medical display device may not receive one or more signals (e.g., video signals, image signals) from the controller device when the controller device is off or in a standby mode. A medical operator may touch the input element to remotely power on the controller device. In some examples, the medical display device may be located in the operating room, thereby allowing the medical operator to power on the controller device without having to leave the operating room and without having to call an IT operator/on-site specialist to power on the controller device.

According to some examples is provided a method for operating a medical display device. The method comprises: displaying an input element on a screen of the medical display device; receiving a user input to the screen; generating a power-on command at the medical display device in response to receiving the user input; and sending the power-on command from the medical display device, wherein the power-on command is used to cause a controller device to power on.

In any of the examples, displaying an input on the screen of the medical display device can comprise displaying the input element in response to not receiving one or more signals from the controller device.

In any of the examples, the medical display device may not receive one or more signals when the controller device is powered off or in standby mode.

In any of the examples, the one or more signals can comprise one or more video signals and/or one or more image signals.

In any of the examples, the method can further comprise: waiting a threshold time period before displaying the input element on the screen of the medical display device.

In any of the examples, the input element can comprise a GUI button, and the user input can comprise a touch on the GUI button.

In any of the examples, the power-on command can comprise a wake on LAN command or a wake on USB command.

In any of the examples, sending the power-on command from the medical display device can comprise: sending the power-on command from the medical display device to a network switch; and sending the power-on command from the network switch to the controller device.

In any of the examples, the method can further comprise: receiving one or more signals from the controller device when the controller device is powered on.

In any of the examples, the method can further comprise: periodically sending a control signal to the controller device.

In any of the examples, the method can further comprise: receiving an acknowledgement signal from the controller device in response to the controller device receiving the control signal.

In any of the examples, the medical display device can be a medical touch screen device and/or the screen of the medical display device can be a touch screen, wherein receiving the user input to the screen can comprise receiving a touch input on the touch screen.

According to some examples, a system comprises: a medical display device comprising a screen configured to: display an input element on the screen, receive a user input to the screen, and generate and send a power-on command in response to receiving the user input; a controller device configured to: receive the power-on command from the medical display device, and power on in response to receiving the power-on command; and a routing system configured to route the power-on command from the medical display device to the controller device.

In any of the examples, the medical display device can be configured to display the input element on the screen when the medical display device has not received one or more signals from the controller device.

In any of the examples, the medical display device has not received one or more signals from the controller device when the controller device is powered off or in standby mode.

In any of the examples, the one or more signals can comprise one or more video signals and/or one or more image signals.

In any of the examples, the medical display device can comprise a medical touch screen device and/or the screen can comprise a touch screen.

In any of the examples, the routing system can comprise a network switch configured to route one or more signals from the controller device to the medical display device.

In any of the examples, the routing system can comprise an encoder configured to encode one or more signals from the controller device.

In any of the examples, the routing system can be configured to route the one or more encoded signals to the medical display device.

In any of the examples, the medical display device can be located in an operating room, and the controller device can be located outside of the operating room.

In any of the examples, the medical display device and the controller device can be located in an operating room.

In any of the examples, the medical display device can be configured to receive one or more signals from the controller device when the controller device is powered on.

In any of the examples, the controller device can comprise an image and video capture device, an edge computing device, one or more mobile devices, and/or one or more computing devices capable of operating in standby mode.

According to some examples, a non-transitory computer-readable storage medium stores a computer program product including non-transitory computer-implementable instructions configured to be executed by one or more processors of a system, wherein executing the instructions causes the system to: display an input element on a screen of a medical display device; receive a user input to the screen; generate a power-on command at the medical display device in response to receiving the user input; and send the power-on command from the medical display device, wherein the power-on command is used to cause a controller device to power on.

According to some examples, a computer program product includes computer-implementable instructions configured to be executed by one or more processors of a system, wherein executing the instructions causes the system to: display an input element on a screen of a medical display device; receive a user input to the screen; generate a power-on command at the medical display device in response to receiving the user input; and send the power-on command from the medical display device, wherein the power-on command is used to cause a controller device to power on.

It will be appreciated that any of the variations, aspects, features, and options described in view of the system apply equally to the method and computer program product, and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1 illustrates an example operating room in a medical environment, according to some aspects.
FIG. 2 illustrates a block diagram of an example system comprising a controller device, input devices, and output devices, according to some aspects.
FIG. 3 illustrates a non-limiting example system configuration comprising a medical display device, a routing system, and a controller device, according to some aspects.
FIG. 4 illustrates an example method for powering on a controller device, according to some aspects.
FIG. 5 illustrates an example touch screen showing an input element for powering on a controller device, according to some aspects.
FIG. 6 illustrates an example computing system used for performing any of the methods and systems described herein, according to some aspects.

### DETAILED DESCRIPTION

Reference will now be made in detail to implementations and various aspects and variations of systems and methods described herein. Although several example variations of the systems and methods are described herein, other variations of the systems and methods may include aspects of the systems and methods described herein combined in any suitable manner having combinations of all or some of the aspects described.

Systems and methods according to the principles described herein can reduce or eliminate the need to have a medical operator leave the operating room and/or the need to call an IT operator/on-site specialist in order to power on a controller device. A convenient and efficient way for powering on a controller device includes remotely powering on the controller device via a medical display device *(e.g.,* a medical touch screen device) located in the operating room. In some aspects, the medical display device may provide an input element *(e.g.,* GUI button) on its screen *(e.g.,* touch screen) when a controller device *(e.g.,* an image and video capture device) is powered off or in standby mode (e.g., low power state, sleep mode). The medical operator may touch the input element on the touch screen of the medical display device. The user input (including, but not limited to, touching the GUI button on the touch screen) may cause the medical display device to generate and send a power-on command *(e.g.,* a wake on LAN command, a wake on USB command) through the routing system to the controller device. The controller device may power on in response to receiving the power-on command.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or medical display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability. Suitable processors include central processing units (CPUs), graphical processing units (GPUs), field-programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

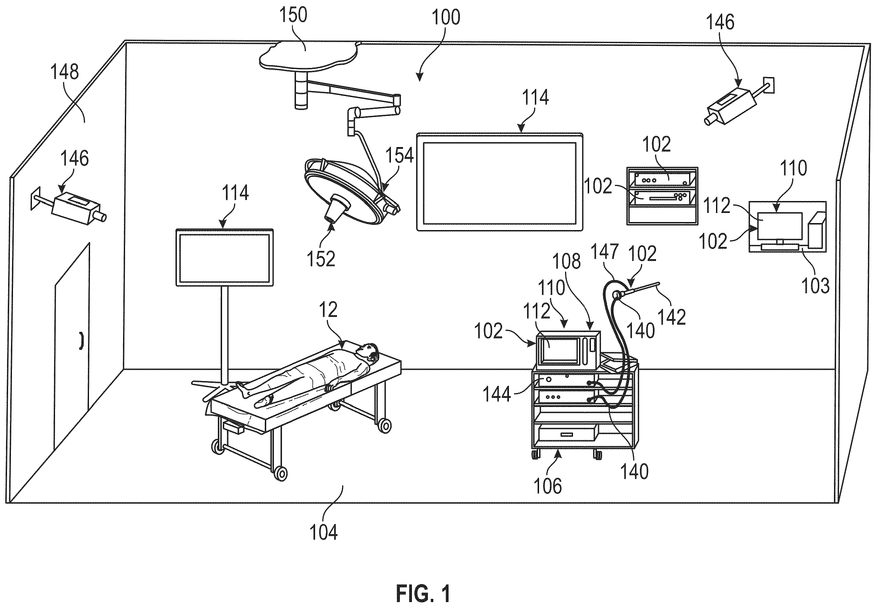

FIG. 1 illustrates an example operating room in a medical environment, according to some aspects. The operating room 100 comprises at least one medical device 102 to assist in performing a medical or surgical procedure and/or for recording keeping purposes. For example, the medical device 102 may be used to input or receive (e.g., from electronic medical records (EMRs), from electronic health records (EHRs), communicated in real-time from another system, etc.) patient information for use with information or images saved onto the medical device 102, displaying information or images from the medical device 102, sending to other medical devices 102, or a combination thereof. In some aspects, the medical device 102 may be used to record patient information, including storing the information or images in an EMR, EHR, or other type of file.

The medical device 102 located within the operating room 100 can include any device that is capable of saving information related to a subject 12. The medical device 102 may or may not be coupled to a network that includes records of the subject 12. The medical device 102 may include a computer system 110 *(e.g.,* a desktop computer, a laptop computer, a tablet device, etc.) having an application server. The computer system 110 can have a motherboard that includes one or more processors or other similar control devices, as well as one or more memory devices. The processor controls the overall operation of the computer system 110 and can include hardwired circuitry, programmable circuitry that executes software, or a combination thereof. The processor may, for example, execute software stored in the memory device. The processor may include, for example, one or more general- or special-purpose programmable microprocessors and/or microcontrollers, application specific integrated circuits (ASICs), programmable logic devices (PLDs), programmable gate arrays (PGAs), or the like. The memory device may include any combination of one or more random access memories (RAMs), read-only memories (ROMs) (which may be programmable), flash memory, and/or other similar storage devices. Patient information may be inputted into the computer system 110 for use with the computer system 110 *(e.g.,* for making an operative note during the medical or surgical procedure on the subject 12 in the operating room 100) and/or the computer system 110 can transmit the patient information to another medical device 102 (via either a wired connection or wirelessly).

The medical device 102 can be positioned in the operating room 100 on a table (stationary or portable), a floor 104, a portable cart 106, an equipment boom, and/or shelving 103. FIG. 1 illustrates two computer systems 110: a first computer system 110 in the form of a desktop computer shelving 103, and a second computer system 110 incorporated into an image and video capture device 108 on a portable cart 106. It is contemplated that the computer system 110 can be on the portable cart 106 *(e.g.,* on the same cart 106 as an image and video capture device 108 or on a separate cart). In some aspects, the image and video capture device 108 and/or associated router(s) (not shown) may be located in a room outside of the operating room 100, such as in a closet. In some other aspects, the image and video capture device 108 and/or associated router(s) (not shown) may be located in a cabinet inside the operating room 100. Further, examples of the disclosure may include any number of computer systems.

The image and video capture device 108 may be capable of recording images, recording videos, displaying images, displaying videos, recording audio, outputting audio, or a combination thereof. In some aspects, patient information can be input into the image and video capture device 108 for adding to the images and videos recorded and/or displayed by the image and video capture device 108. The image and video capture device 108 can include internal storage (e.g., a hard drive, a solid state drive, etc.) for storing the captured images and videos. The image and video capture device 108 can also display any captured or saved images (e.g., from the internal hard drive) or on an associated touchscreen medical display device 112 and/or an additional medical display device 114 coupled to the image and video capture device 108 via either a wired connection or wirelessly. It is contemplated that the image and video capture device 108 could obtain or create images of the subject 12 during a medical or surgical procedure from a variety of sources (e.g., from video cameras, video cassette recorders, X-ray scanners (which convert X-ray films to digital files), digital X-ray acquisition apparatus, fluoroscopes, computed tomography (CT) scanners, magnetic resonance imaging (MRI) scanners, ultrasound scanners, charge-coupled (CCD) devices, and other types of scanners (handheld or otherwise)). If coupled to a network, the image and video capture device 108 can also communicate with a picture archiving and communication system (PACS), as is well known to those skilled in the art, to save images and video in the PACS and for retrieving images and videos from the PACS. The image and video capture device 108 can couple and/or integrate with, e.g., an electronic medical records database and/or a media asset management database.

The image and video capture device 108 is capable of displaying images and videos on a touchscreen medical display device 112 and/or on an additional medical display device 114 captured live by cameras (e.g., a video camera 140 coupled to an associated endoscope 142, which communicates with a camera control unit (CCU) 144 via a fiber optic cable 147, with the camera control unit 144 communicating via wires or wirelessly with the image and video capture device 108) and/or replayed from recorded images and videos. It is further contemplated that the image and video capture device 108 can display images and videos on the touchscreen medical display device 112 and/or on the additional medical display device 114 captured live by a room camera 146 fixed to walls 148 or a ceiling 150 of the operating room 100 *(e.g.,* a room camera 146 as shown, or a camera 152 in an overhead light 154). In some aspects, the image and video capture device 108 can be used to integrate, annotate, and/or correct images.

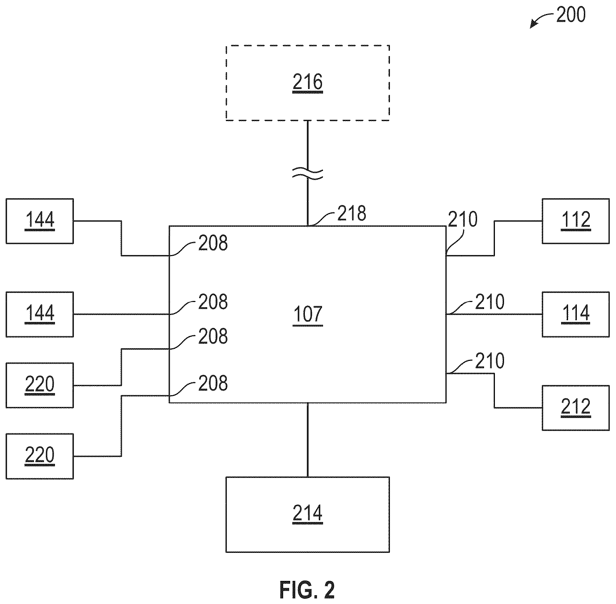

FIG. 2 illustrates a block diagram of an example system 200 comprising a controller device 107, input devices, and output devices, according to some aspects. The controller device 107 receives one or more signals to be routed to one or more medical display devices 112 or 114. The one or more signals may comprise video signals and/or image signals (data) associated with treatment of a patient (e.g., subject 12 shown in FIG. 1). The signals can comprise images and/or videos generated during treatment of the patient in support of one or more medical procedures, such as video captured by an endoscopic camera. Example controller devices 107 include, but are not limited to an image and video capture device 108, an edge computing device, one or more mobile devices, one or more computing devices capable of operating in standby mode, etc. Examples of source devices include, without limitation, endoscopic systems, open field imaging systems, x-ray systems such as intraoperative c-arm systems, computer tomography (CT) systems, ultrasound systems, magnetic resonance imaging (MRI) systems, and nuclear medicine systems.

In some aspects, the controller device 107 may receive data from one or more non-imaging devices 220 that may be used in connection with (e.g., during) a medical imaging session (e.g., surgical procedure) and may provide information that may be relevant for display during a medical imaging session. Non-limiting examples of non-imaging devices include insufflators, illumination controllers, and voice control systems.

The controller device 107 may receive a signal (e.g., video signal, image signal) from the one or more CCUs 144 through one or more input ports 208. The controller device 107 generates one or more display feeds using the received signal(s) and transmits the one or more display feeds to one or more medical display devices 112 or 114 via one or more output ports 210. For example, the controller device 107 may generate a display feed that includes enhanced imaging of tissue of a patient based on imaging generated by one or more CCUs 144, and the enhanced imaging may be displayed on one or more medical display devices 112 or 114 to assist a practitioner during treatment of the patient. In some aspects, the controller device 107 can operate with a router to route signals from the input port(s) 208 to the output port(s) 210. The format of the data included in the signal may include analog, digital, HD format, UHD format *(e.g.,* 4K or 8K video), or the like. In some aspects, the controller device 107 may be configured to control a signal router or a network router that converts the signal to an IP multimedia stream (e.g., using encoders and decoders). In some aspects, the signal router may be capable of routing signals without use of a network router and does not require conversion to an IP multimedia stream. The controller device 107, signal router, or both may operate with (e.g., integrated with or located externally) a circuit that switches between multimedia inputs for a given multimedia output.

Controller device 107 may also transmit display feeds to one or more recording devices 212 for recording enhanced imaging for later retrieval. Input ports 208 and output ports 210 may be any suitable type of data transmission ports, such as digital visual interface (DVI) ports, high-definition multimedia interface (HDMI) ports, DisplayPort (DP) ports, VGA ports, RS232 ports, IP (network, e.g., Ethernet) ports, and the like.

Controller device 107 may be coupled to one or more networks 216 via one or more network connections 218. The one or more networks 216 may include a local network such as a hospital information system, or a wider network such as a wide area network or the internet. A network connection 218 can be a wired connection, such as an Ethernet connection, or a wireless network connection, such as a Wi-Fi connection. In some aspects, the controller device 107 may access the one or more networks 216 to retrieve configuration data stored at a network location for configuring the controller device 107 for an imaging session, and/or may access the one or more networks to receive updated software and/or updated hardware files for processing imaging data. In some aspects, the controller device 107 may access a database comprising information, such as EMRs, EHRs, or other patient data, for retrieval and/or storage.

One or more user interfaces 214 may be in communication with (e.g., connected to) the controller device 107 for a user to provide input to the controller device 107. The user may input data related to configuring the controller device 107 for an imaging session. User input can include, for example, selection of a practitioner profile associated with an upcoming imaging session, selection of the type of imaging session or types of procedure to be performed during an imaging session, selection of which inputs (e.g., multimedia inputs) are routed to which outputs *(e.g.,* multimedia outputs), predetermined routing selections *(e.g.,* surgeon- or patient-specific, preset configurations), or any other relevant information such as the operation mode and associated parameters for the medical display device 112 or 114. The one or more user interfaces 214 may include a tablet, a keyboard, a mouse, a voice control system, a keypad, a touchscreen, or any combination thereof. In some aspects, the one or more user interfaces 214 may include one or more indicators, such as a signal input indicator (e.g., phase-locked loop (PLL) indicator) that indicates when an input port 208 comprises a signal.

In some aspects, the controller device 107 processes received medical imaging data and any other relevant data and generates enhanced display feeds for display on one or more medical display devices 112 or 114 during an imaging session. According to some aspects, the controller device 107 may combine multiple imaging sources into a single display feed, process received imaging data to generate richer imaging data, modify imaging data for better utilization of display space, and/or reconfigure the processing of imaging data depending on the needs and preferences of users from imaging session to imaging session.

Aspects of the disclosure include one or more systems and methods for powering on a controller device by a medical display device. As a non-limiting example, in some aspects, the controller device may be located away (e.g., within 75 feet (or 22.55 meters), within 328 feet (or 100 meters)) from the operating room, and the medical display device may be located within the operating room. In some aspects, the controller device may be in a remote location, such as in a closet outside of the operating room, and the medical display device may be located within the operating room. In some aspects, the controller device and medical display device may be located within, but at different locations of, the operating room. A medical operator may power down one or more medical devices when the operating room is not used for a long period of time, such as at the end of the day, and the medical operator may power on a controller device once use of the operating room resumes. It can create a risk of unsanitary conditions and/or it may be burdensome for the medical operator to power on a controller device. If the controller device is not powered on, the medical operator may have to leave the operating room to power on the controller device, creating a sanitary risk and inefficiencies by delaying the medical procedure. Additionally or alternatively, the medical procedure may have to be delayed in order to gain access to the controller device (e.g., located in a locked closet, located in a hard-to-access location).

Examples of the disclosure allows a medical operator to power on the controller device remotely using, *e.g.,* a medical display device. By having the ability to power on the controller device remotely, the medical operator may not have to leave the operating room and/or incur delays in order to power on the controller device. Examples of the disclosure may additionally or alternatively provide the ability to power off the controller device remotely. By conveniently and efficiently providing the ability to power off the controller device remotely, medical facilities (e.g., hospitals, surgical centers) may be more likely to incorporate power down procedures when the operating room is not used for a long period of time, such as at the end of the day. Periodic power cycles may help eliminate or reduce errors and process updates, thereby increasing the overall reliability of the medical devices.

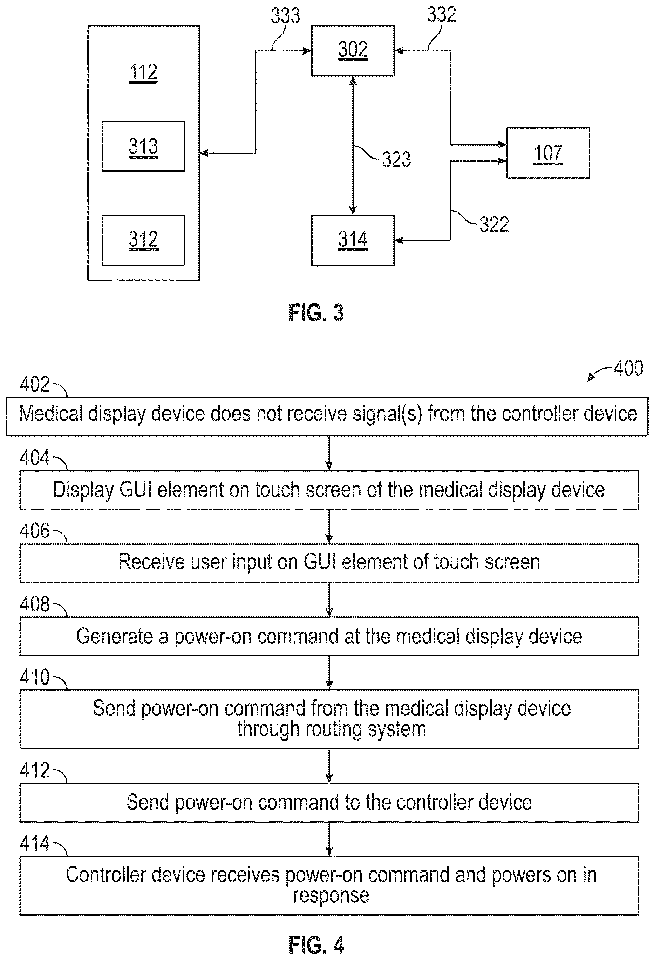

FIG. 3 illustrates a non-limiting example system configuration comprising a medical display device, a routing system, and a controller device, according to some aspects. The medical display device 112 may be coupled to a controller device 107 via routing system 302. The routing system 302 may comprise a network switch, for example, configured to route one or more video or image signals 322 to/from the controller device 107. The network switch may additionally or alternatively be configured to route one or more data signals 332 to/from the controller device 107. The medical display device 112 may be configured to receive a video or image signal 322 from the controller device 107. In some examples, the medical display device 112 may include a microcontroller 313 and/or a decoder 312 integrated into the medical display device 112. The controller device 107 may be configured to send the video or image signal 322 to an encoder 314. The encoder 314 may be configured to encode the video or image signal 322 and send the encoded video or image signal 323 to the medical display device 112 via the routing system 302. In some aspects, one or more data signals 332 may be communicated between the medical display device 112 and the controller device 107. As discussed in more detail below, the signal(s) 332 may comprise a control signal and an acknowledgement signal, for example. In some aspects, the medical display device 112 may send a power-on command 333 (as discussed in more detail below).

Examples of the disclosure may comprise one or more methods (and associated systems) for powering on a controller device remotely (e.g., using a device external from the controller device). Allowing the controller device to be powered on remotely may minimize the burden and delays associated with powering on a controller device that is located outside of the operating room or located in a hard-to-access location within the operating room. Allowing the controller device to be powered on remotely may also minimize the risk of unsanitary conditions.

FIG. 4 illustrates an example method for powering on a controller device, according to some aspects. The method 400 may comprise the medical display device 112 (including the microcontroller 313, decoder 312, or both) not receiving one or more signals from a controller device 107 at step 402. For example, the controller device 107 may be off or in standby mode (such as in a low power state or sleep mode), and as a result, may not be sending signals to the medical display device 112.

The medical display device 112 may display an input element *(e.g.,* a GUI button on the touch screen) of the medical display device 112 in step 404, for example, due to not receiving video. The medical display device 112 may wait a threshold time period before providing the input element (for example, displaying a GUI button on the touch screen of the medical display device 112). Example threshold time periods can include, but are not limited to, 20 seconds, 25 seconds, 30 seconds, etc.

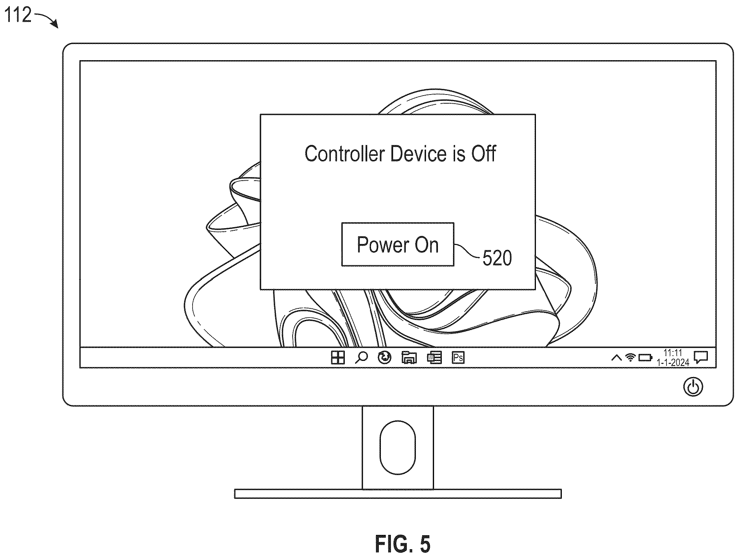

In some aspects, the input element may comprise a portion or all of the touch screen. For example, the input element may be a GUI element such as GUI button 520, as shown in FIG. 5. The touch screen of the medical display device 112 may display a message (e.g., "controller device is off") and the GUI button 520. The GUI button 520 may comprise a message such as "power on" that is touch sensitive.

Returning to FIG. 4, in step 406, a medical operator may provide user input on the input element of the touch screen. For example, the medical operator may touch the GUI button 520 (of FIG. 5). The medical display device 112 may receive the user input to the screen in step 406. The medical display device 112 may generate a power-on command 333 in response to receiving the user input (step 408). Example power-on commands include, but are not limited to, a wake on LAN command and/or a wake on USB command.

In step 410, the medical display device 112 may send the power-on command 333 to the routing system 302. The routing system 302 may comprise a network switch configured to route signals to medical devices, for example. The routing system 302 (e.g. network switch) may send the power-on command to the controller device 107 (step 412). In step 414, the controller device 107 may receive the power-on command and turn on in response. In some aspects, once on, the controller device 107 may send one or more video or image signals 322 to the encoder 314. The encoder 314 may encode the video or image signals 322 and send the encoded signals 323 to the medical display device 112 via the routing system 302.

In some aspects, the medical display device 112, the decoder 312, and/or the microcontroller 313 may not receive one or more video or image signals 322 from the controller device 107, but the controller device 107 may be powered on. For example, the controller device 107 may be on, but not coupled to the medical display device 112. The controller device 107 may not be coupled to the medical display device 112 when the controller device 107 is not connected to the network switch of the routing system 302 and/or the routing system 302 is not connected to the medical display device 112, for example. The decoder 312 and/or microcontroller 313 may periodically send a control signal to the controller device 107, and the controller device 107 can acknowledge receipt of the control signal by responding with an acknowledgement signal (in response to receiving the control signal). The acknowledgement signal may be used as an indication that the controller device 107 is on. If the controller device 107 is on, but the decoder 312 and/or microcontroller 313 is not receiving one or more video and/or image signals 322 from the controller device 107, then the decoder 312 and/or microcontroller 313 and controller device 107 may be decoupled, or the controller device 107 may be performing other functions (e.g., functions that do not involve sending one or more video and/or image signals 322 to the medical display device 112). Examples of the disclosure may also include the controller device 107 periodically sending a control signal to the decoder 312, and the decoder 312 responding with an acknowledgement signal.

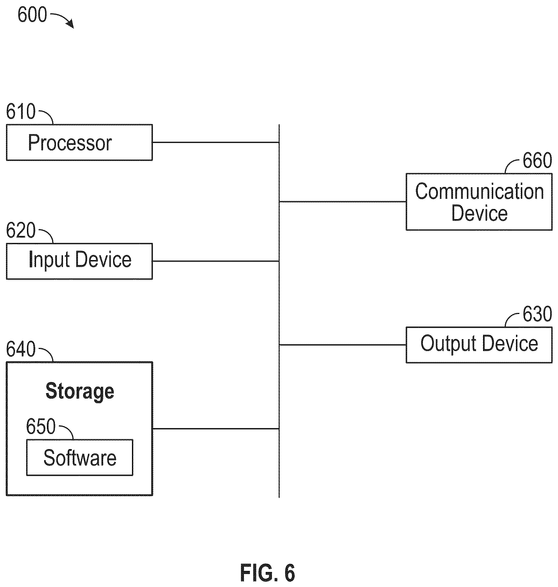

FIG. 6 illustrates an example computing system, in accordance with some examples, that can be used for performing any of the methods described herein, including method 400 of FIG. 4, and can be used for any of the systems described herein, including the medical display device 112, the controller device 107, and/or a medical device 102. System 600 can be a computer coupled to a network, which can be, for example, an operating room network or a hospital network. System 600 can be a client computer or a server. As shown in FIG. 6, system 600 can be any suitable type of controller (including a microcontroller) or processor (including a microprocessor) based system, such as an embedded control system, personal computer, workstation, server, or handheld computing device (portable electronic device) such as a phone or tablet. The system can include, for example, one or more of processor 610, input device 620, output device 630, storage 640, or communication device 660.

Input device 620 can be any suitable device that provides input, such as a touch screen, keyboard or keypad, mouse, gesture recognition component of a virtual/augmented reality system, or voice-recognition device. Output device 630 can be or include any suitable device that provides output, such as a touch screen, haptics device, virtual/augmented reality display, or speaker.

Storage 640 can be any suitable device that provides storage, such as an electrical, magnetic, or optical memory including a RAM, cache, hard drive, removable storage disk, or other non-transitory computer readable medium. Communication device 660 can include any suitable device capable of transmitting and receiving signals over a network, such as a network interface chip or device. The components of the computer can be coupled in any suitable manner, such as via a physical bus or wirelessly.

Software 650, which can be stored in storage 640 and executed by processor 610, can include, for example, the programming that embodies the functionality of the present disclosure *(e.g.,* as embodied in the devices as described above). For example, software 650 can include one or more programs for performing one or more of the steps of the methods disclosed herein.

Software 650 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 640, that can contain or store programming for use by or in connection with an instruction execution system, apparatus, or device.

Software 650 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic, or infrared wired or wireless propagation medium.

System 600 may be coupled to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 600 can implement any operating system suitable for operating on the network. Software 650 can be written in any suitable programming language, such as C, C++, C#, Java, or Python. In various examples, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for operating a medical display device, the method comprising:
displaying an input element on a screen of the medical display device;
receiving a user input to the screen;
generating a power-on command at the medical display device in response to receiving the user input; and
sending the power-on command from the medical display device, wherein the power-on command is used to cause a controller device to power on.

2. The method of claim 1, wherein displaying an input on the screen of the medical display device comprises displaying the input element in response to not receiving one or more signals from the controller device,
wherein the medical display device is not receiving one or more signals when the controller device is powered off or in standby mode.

3. The method of claim 2, wherein the one or more signals comprise one or more video signals and/or one or more image signals.

4. The method of any of claims 1-3, further comprising:
waiting a threshold time period before displaying the input element on the screen of the medical display device.

5. The method of any of claims 1-4, wherein the power-on command comprises a wake on LAN command or a wake on USB command.

6. The method of any of claims 1-5, wherein sending the power-on command from the medical display device comprises:
sending the power-on command from the medical display device to a network switch; and
sending the power-on command from the network switch to the controller device.

7. The method of any of claims 1-6, further comprising:
periodically sending a control signal to the controller device; and
receiving an acknowledgement signal from the controller device in response to the controller device receiving the control signal.

8. A system comprising:
a medical display device comprising a screen configured to:
display an input element on the screen,
receive a user input to the screen, and
generate and send a power-on command in response to receiving the user input;
a controller device configured to:
receive the power-on command from the medical display device, and
power on in response to receiving the power-on command; and
a routing system configured to route the power-on command from the medical display device to the controller device.

9. The system of claim 8, wherein the medical display device is configured to display the input element on the screen when the medical display device has not received one or more signals from the controller device; and
wherein the medical display device has not received one or more signals from the controller device when the controller device is powered off or in standby mode.

10. The system of claim 8 or 9, wherein the one or more signals comprise one or more video signals and/or one or more image signals.

11. The system of claim 8, 9 or 10, wherein the medical display device comprises a medical touch screen device and/or the screen comprises a touch screen.

12. The system of any of claims 8-11, wherein the routing system comprises a network switch configured to route one or more signals from the controller device to the medical display device; and/or wherein the routing system comprises an encoder configured to encode one or more signals from the controller device, wherein the routing system is configured to route the one or more encoded signals to the medical display device.

13. The system of any of claims 8-12, wherein the medical display device is configured to receive one or more signals from the controller device when the controller device is powered on.

14. The system of any of claims 8-13, wherein the controller device comprises an image and video capture device, an edge computing device, one or more mobile devices, or one or more computing devices capable of operating in standby mode.

15. A computer program product including computer-implementable instructions configured to be executed by one or more processors of a system, wherein executing the instructions causes the system to:
display an input element on a screen of a medical display device;
receive a user input to the screen;
generate a power-on command at the medical display device in response to receiving the user input; and
send the power-on command from the medical display device, wherein the power-on command is used to cause a controller device to power on.
